# EUROPEAN PATENT APPLICATION

(11) **EP 2 022 857 A2**
(43) Date of publication of application: **11.02.2009**
(21) Application number: 08170135.1
(22) Date of filing: 28.02.2007
(51) Int. Cl.: C12Q 1/00, C12Q 1/60, G01N 27/327, G01N 33/92

(54) **Sensing member for detecting total cholesterol of blood sample**

(62) Divisional of application: 07004177.7
(71) Applicant: General Life Biotechnology Co., Ltd., Jhongsiao E. Rd. Taipei City 106 (TW)
(72) Inventor: Su, Chein-Shyong, Taipei City 106 (TW); Hung, Miao-Ling, Taipei City 106 (TW); Jing, Ning-Jun, Taipei City 106 (TW); Wu, Tai-Guang, Taipei City 106 (TW)
(74) Representative: Epping - Hermann - Fischer

(57) **Abstract**

A sensing member for detecting total cholesterol of a blood sample is disclosed, which includes: a working electrode and a counter electrode respectively formed on a first substrate and a second substrate, wherein, an opening is formed in one of the first and second substrates corresponding in position to the electrode for allowing the blood sample to pass through; a dielectric layer formed on one of the surfaces of the first and second substrates and exposing the electrode and end connector such that the end connector can be electrically connected to an electronic device used for applying electric voltage; a reactive pad disposed between the working electrode and the counter electrode and containing an reactive reagent, wherein the reactive reagent comprises an electron transport material, an enzyme capable of reacting with the analyte and generating a current corresponding to the concentration, and a buffer solution; and a separating pad disposed on the opening for removing interfering materials from the blood sample before the blood sample enters into the opening. By disposing the separating pad at a specified position, corpuscles and interfering giant molecules can be removed from the blood sample so as to quickly and accurately detect total cholesterol of the whole blood sample.

## Description

### BACKGROUND OF THE INVENTION

### 1. Field of the Invention:

The present invention relates generally to a sensing member for detecting total cholesterol of blood sample, and more particularly to a sensing member that detects total cholesterol of blood sample through an electrochemical method.

### 2. Description of Related Art:

Total cholesterol level in blood constitutes an important part of a lipid profile, which is generally a primary focus in preventing and/or curing diseases such as arteriosclerosis and cardiovascular diseases. If cholesterol level in blood can be detected conveniently in our daily life so as to timely control the cholesterol in blood within a standard value, probability of occurrence of arteriosclerosis and cardiovascular diseases will be effectively reduced.

Currently, blood cholesterol level can be detected through a biochemical molecular level detection method, which uses spectrophotometric instrumentation combined with assay of chromogenic reaction of reactive composition at specified wavelength such as light absorption. However, a spectrophotometric method is considered to give a qualitative or semi-quantitative detection result and such instrumentation must be used in specified locations such as in a laboratory, which is rather inconvenient. Meanwhile, this method has drawback of instability in determining biochemical molecules of low concentration.

An electrochemical detecting method is a rapid and reliable approach, which uses a conducting electrode to detect electrical current positively proportional to the concentration of analyte of the sample transported by electrons generated in a biochemical reaction through an enzyme. As an electrode detecting sheet is easy to produce, has a low cost and can be conveniently transported, it has been widely applied in various kinds of biochemical detectors for detecting such as blood sugar, uric acid and cholesterol.

Generally, blood samples used in cholesterol level detection needs to be preprocessed through several steps such as centrifuging, depositing and separating such that materials such as corpuscles that may interfere the detecting result can be removed. However, as such preprocessing steps cannot be performed at home, a whole blood sample must be used in cholesterol level detection. US Patent No.5,695,947 discloses a biosensor used for detecting cholesterol level, wherein blood serum is used as a detecting sample. However, the patent does not disclose how the corpucles of the whole blood sample and other interfering materials will affect the detecting result.

US Patent No.6,033,866 discloses a biosensor which performs blood sugar detection based on an electrochemical assay principle. According to this patent, when a sample is dripped into a reactive pad located in an interlayer through an opening located on the reference electrode, corpuscles and interfering materials are separated from the sample through a separating film. However, cholesterol detection is different from blood sugar detection. Particularly, compared with adverse effect of interfering compositions on blood sugar detection, adverse effect of interfering compositions of a whole blood sample on the cholesterol detection can be much more serious. In addition, said patent does not disclose how position of the separating film will affect the detection result.

US Patent Publication No.2003/0183591 discloses a biosensor capable of separating corpuscles from a whole blood sample. When a whole blood sample enters into a filtering area, flowing rate of corpuscles is decreased according to the principle of horizontal chromatography, thus allowing the plasma to reach the electrode reaction area for detection. However, the horizontal chromatography needs a long sample separating time. Further, the flow rate of the corpuscles is difficult to be controlled, and it is impossible to completely keep the corpuscles from entering into the reaction area. On the other hand, Japan Publication No.2004-245736 discloses a detecting sheet capable of using a whole blood sample to detect HDL cholesterol and triglycerol. However, the test sheet has a complex structure, and a corpuscle delay agent is needed. In addition, filtering of corpuscles and flowing of plasma depend on an additional pumping mechanism. Therefore, as much as several tens of microliter samples are needed and reactive time can reach several tens of minutes.

Therefore, there is a need to provide a sensor that can directly use whole blood sampled for detection and can rapidly and accurately obtain detection results.

### SUMMARY OF THE INVENTION

According to the above drawbacks, an objective of the present invention is to provide a sensing member capable of quickly detecting total cholesterol of blood samples.

Another objective of the present invention is to provide a sensing member capable of accurately detecting total cholesterol of blood samples.

A further objective of the present invention is to provide a sensing member capable of detecting total cholesterol of whole blood samples.

In order to attain the above and other objectives, the present invention discloses a sensing member for detecting total cholesterol of a blood sample, which comprises: a working electrode and a counter electrode respectively formed on a first substrate and a second substrate, wherein, an opening is formed in one of the first and second substrates corresponding in position to the electrode for allowing the blood sample to pass through; a dielectric layer formed on one of the surfaces of the first and second substrates and exposing the electrode and end connector such that the end connector can be electrically connected to an electronic device used for applying electric voltage; a reactive pad disposed between the working electrode and the counter electrode and comprising an reactive reagent, wherein the reactive reagent comprises an electron transport material, an enzyme capable of reacting with the analyte and generating a current corresponding to the concentration, and a buffer solution; and a separating pad disposed on the opening for removing interfering materials from the blood sample before the blood sample enters into the opening. By using the separating pad disposed on the opening, interfering materials of the blood sample can be removed from the blood sample before the blood sample enters into the opening, thereby quickly and accurately detecting total cholesterol of whole blood samples.

Another sensing member for detecting total cholesterol of a blood sample according to the present invention comprises: a working electrode and a counter electrode respectively formed on a first substrate and a second substrate; a dielectric layer formed on one of the surfaces of the first and second substrates and exposing the electrode and end connector such that the end connector can be electrically connected to an electronic device used for applying electric voltage; a reactive pad disposed between the working electrode and the counter electrode and comprising an reactive reagent, wherein the reactive reagent comprises an electron transport material, an enzyme capable of reacting with the analyte and generating a current corresponding to the concentration, and a buffer solution; and a separating pad disposed between the first and second substrates, wherein the blood sample is passed through the separating pad such that interfering materials can be separated and removed from the blood sample and then the blood sample is laterally diffused to the reactive pad. By using the separating pad disposed between the first and second substrates, interfering materials of the blood sample can be removed from the blood sample. Thereafter, the blood sample is laterally diffused to the reactive pad for total cholesterol detection. Thus, the sensing member of the present invention can quickly and accurately detect total cholesterol of whole blood samples.

### BRIEF DESCRIPTION OF DRAWINGS

FIGS.1 is an exploded diagram of a sensing member for detecting total cholesterol of blood according to a first embodiment of the present invention;
FIGS.2 is an exploded diagram of a sensing member for detecting total cholesterol of blood according to a second embodiment of the present invention;
FIG.3 is an exploded diagram of a sensing member for detecting total cholesterol of blood according to a third embodiment of the present invention; and
FIG.4 is an exploded diagram of a sensing member for detecting total cholesterol of blood according to a fourth embodiment of the present invention.

### DETAILED DESCRIPTION OF PREFERRED EMBODIMENTS

The following illustrative embodiments are provided to illustrate the disclosure of the present invention, these and other advantages and effects can be apparent to those skilled in the art after reading the disclosure of this specification. The present invention can also be performed or applied by other different embodiments. The details of the specification may be on the basis of different points and applications, and numerous modifications and variations can be made without departing from the spirit of the present invention.

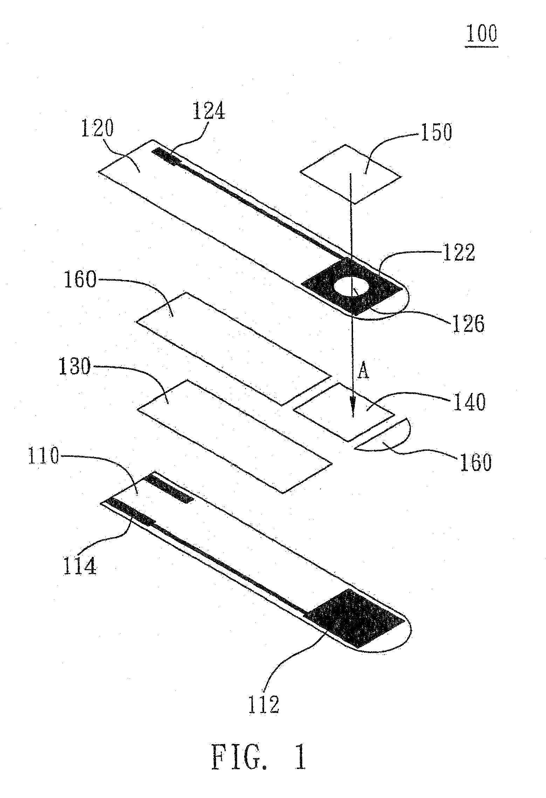

FIG.1 shows a sensing member for detecting total cholesterol of blood according to the present invention. As shown in FIG.1, the sensing member 100 comprises a first substrate 110, a second substrate 120, a dielectric layer 130, a reactive pad 140, and a separating pad 150. The substrates can be made of dielectric polymer, SiO₂, or AlO₂. Therein, the dielectric polymer can be, but not limited to PVC, polystyrene, polyester, polycarbonate, polyether, polyethylene, polypropylene and PET. In the present embodiment, PET sheet substrates are used as the first and second substrates 110, 120. Through a screen printing method, a working electrode 112 and metallic conducting lines 114 are formed on the first substrate 110, and a counter electrode 122 and metallic conducting lines 124 are formed on the second substrate 120. The second substrate 120 has an opening 126 formed at a position corresponding to the counter electrode 122 for allowing the blood sample to pass through. The working electrode and the counter electrode may be made of carbon electrode, graphite electrode, or metal particle. Preferably, the working electrode and the counter electrode are made of carbon electrode. The metallic conducting lines may be made of silver, gold or platinum. Preferably, the metallic conducting lines are made of silver.

A dielectric layer 130 is formed on surface of the first substrate 110 so as to cover the surface thereof. Therein, the working electrode 112 and the electrode end connector are exposed from the dielectric layer 130 such that the end connector can be electrically connected to an electronic device used for applying electric voltage. An adhesive layer 160 has an empty location formed corresponding to the working electrode 112 and the counter electrode 122. The reactive pad 140 comprising reactive reagent is disposed in the empty location of the adhesive layer 160 and then, the first substrate 110 with the working electrode 112 and the dielectric layer 130 and the second substrate 120 with the counter electrode 122 are attached together face by face, thereby disposing the reactive pad 140 comprising the reactive reagent between the working electrode 112 of the first substrate 110 and the counter electrode 122 of the second substrate 120.

The reactive reagent of the reactive pad 140 comprises an electron transport material, an enzyme capable of reacting with the analyte and generating a current corresponding to the concentration of the analyte, and a buffer solution. The electron transport material can be, but not limited to ferricyanate such as potassium ferricyanide, ferrocene, para-benzoquinone, phenazine methosulfate, Indophenol, TMB(3,3',5,5'-Tetramethylbenzidine) and β-naphthoquinone-4-potassium sulfonate. The enzyme capable of reacting with the analyte and generating a current corresponding to the concentration comprises cholesterol esterase, cholesterol oxidase and catalase. The buffer solution can be phosphate buffer solution, TRIS buffer solution or MES buffer solution. In the present embodiment, the reactive pad 140 can further comprise a surface active reagent such as Triton X-100 for improving solubility of ester cholesterol. Dripping the reactive reagent to the reactive pad 140 and roasting it 30 minutes at 40°C,a reactive pad 140 comprising the reactive reagent can be formed.

Subsequently, the separating pad 150 such as a glass fiber filter paper is disposed on the opening 126 of the second substrate 120. Thus, the sensing member 100 of the first embodiment of the present invention is formed. The separating pad 150 is used to remove interfering materials from the blood sample before the blood sample enters into the reactive pad 140 through the opening 126 in direction A as shown in FIG.1. Particularly, the separating pad of the present invention is capable of removing corpuscles and interfering materials from a whole blood sample, thus quickly and accurately obtaining detection results.

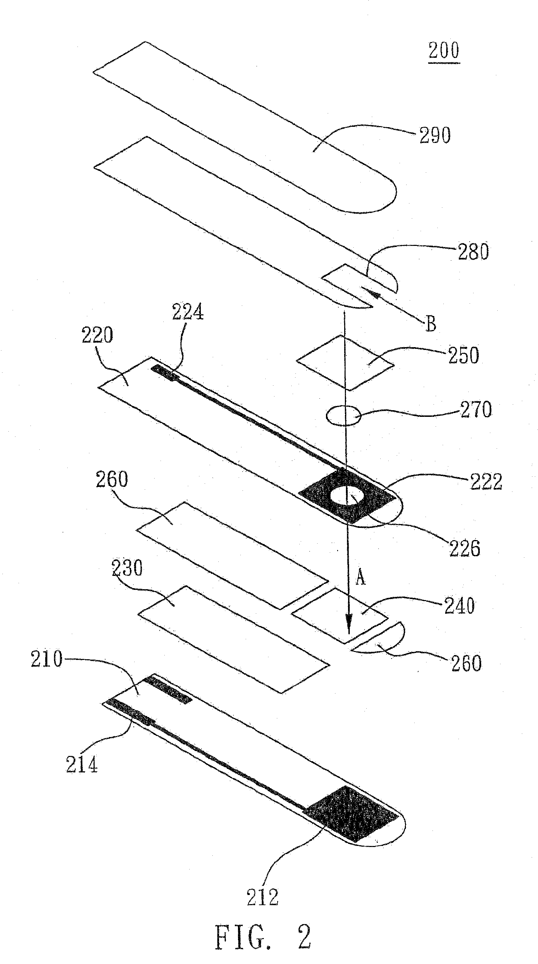

FIG.2 shows a sensing member for detecting total cholesterol of blood according to a second embodiment of the present invention. As shown in FIG.2, the sensing member 200 comprises a first substrate 210, a second substrate 220, a dielectric layer 230, a reactive pad 240, a separating pad 250, a flow conducting sheet 270, a capillary introducing area 280, and an upper covering sheet 290. Through a screen print method, a working electrode 212 and metallic conducting lines 214 are formed on the first substrate 210, and a counter electrode 222 and metallic conducting lines 224 are formed on the second substrate 220. The second substrate 220 has a circular opening 226 formed at a position corresponding to the counter electrode 222 for allowing the blood sample to pass through.

A dielectric layer 230 is formed on surface of the first substrate 210 so as to cover the surface thereof. Therein, the working electrode 212 and the electrode end connector are exposed from the dielectric layer 230 such that the end connector can be electrically connected to the electronic device used for applying electric voltage. An adhesive layer 260 has an empty location formed corresponding to the working electrode 212 and the counter electrode 222. The reactive pad 240 comprising reactive reagent is disposed on the empty location of the adhesive layer 260 and then, the first substrate 210 with the working electrode 212 and the dielectric layer 230 and the second substrate 220 with the counter electrode 222 are attached together face by face, thereby disposing the reactive pad 240 comprising the reactive reagent between the working electrode 212 of the first substrate 210 and the counter electrode 222 of the second substrate 220.

Subsequently, the flow conducting sheet 270 and the separating pad 250 are disposed in sequence on the circular opening 226 of the second substrate 220. In the present embodiment, the flow conducting sheet 270 substantially has a same outline as the circular opening 226, which conducts the blood sample from the separating pad 250 through the circular opening 226 into the reactive pad 240 in direction A as shown in FIG.2. The capillary introducing area 280 is disposed above the separating area 250 so as to smoothly conduct the blood sample into the separating pad 250 in direction B as shown in FIG.2. Finally, the upper covering sheet 290 covers the said structure, thus forming a sensing member according to the second embodiment of the present invention.

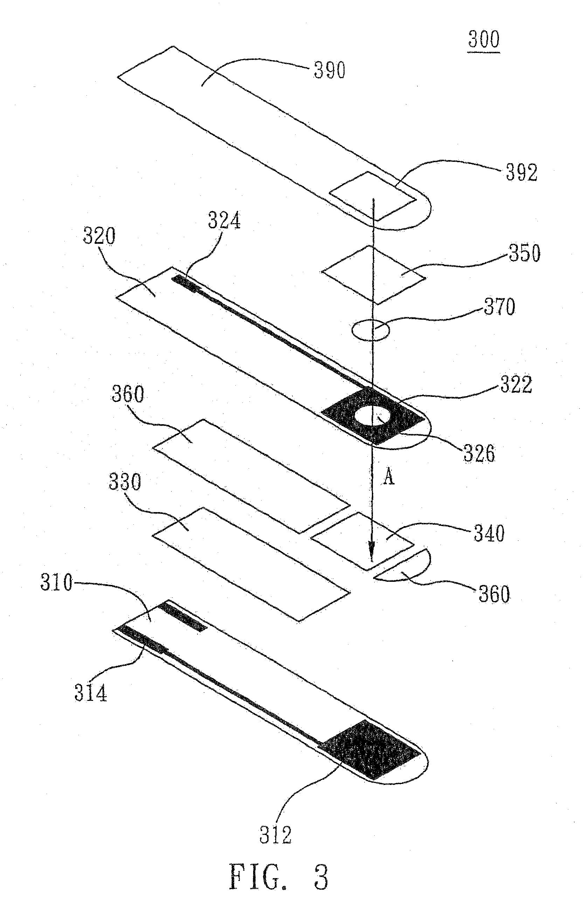

FIG.3 shows a sensing member for detecting total cholesterol of blood according to a third embodiment of the present invention. As shown in FIG.3, the sensing member 300 comprises a first substrate 310, a second substrate 320, a dielectric layer 330, a reactive pad 340, a separating pad 350, a flow conducting sheet 370, and an upper covering sheet 390. Through a screen print method, a working electrode 312 and metallic conducting lines 314 are formed on the first substrate 310, and a counter electrode 322 and metallic conducting lines 324 are formed on the second substrate 320. The second substrate 320 has a circular opening 326 formed at a position corresponding to the counter electrode 322 for allowing the blood sample to pass through.

A dielectric layer 330 is formed on surface of the first substrate 310 so as to cover the surface thereof. Therein, the working electrode 312 and the electrode end connector are exposed from the dielectric layer 330 such that the end connector can be electrically connected to the electronic device used for applying electric voltage. An adhesive layer 360 has an empty location formed corresponding to the working electrode 312 and the counter electrode 322. The reactive pad 340 comprising reactive reagent is disposed on the empty location of the adhesive layer 360 and then, the first substrate 310 with the working electrode 312 and the dielectric layer 330 and the second substrate 320 with the counter electrode 322 are attached together face by face, thereby disposing the reactive pad 340 comprising the reactive reagent between the working electrode 312 of the first substrate 310 and the counter electrode 322 of the second substrate 320.

Subsequently, the flow conducting sheet 370 and the separating pad 350 are disposed in sequence on the circular opening 326 of the second substrate 320, and then the upper covering sheet 390 is used to cover the said structure, thus forming a sensing member of the third embodiment of the present invention. Therein, the upper covering sheet 390 has an inlet 392 formed corresponding in position to the separating pad 350. The blood sample is injected into the sensing member through the inlet 392, then the blood sample flows through the separating pad 350, the flow conducting sheet 370 and the circular opening 326 and enters into the reactive pad 340 in direction A as shown in FIG.3.

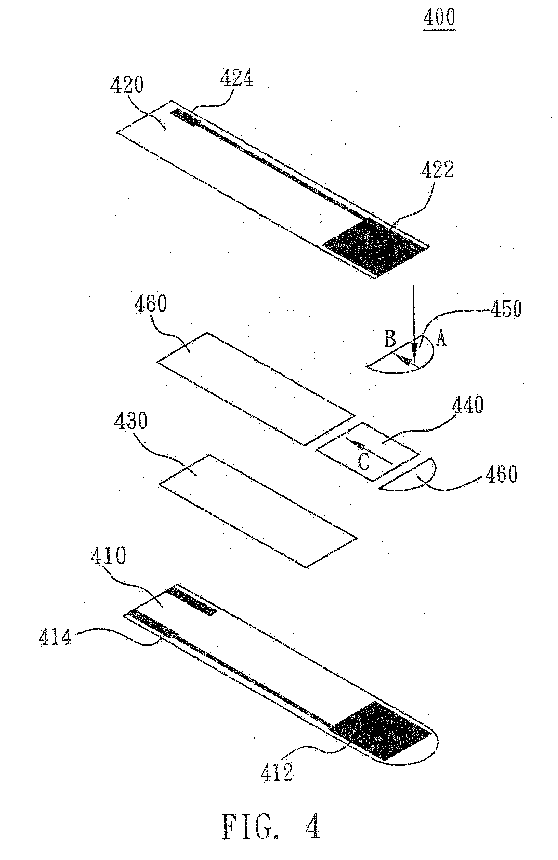

FIG.4 shows a sensing member for detecting total cholesterol of blood according to a fourth embodiment of the present invention. As shown in FIG.4, the sensing member 400 comprises a first substrate 410, a second substrate 420, a dielectric layer 430, a reactive pad 440, and a separating pad 450. Through a screen print method, a working electrode 412 and metallic conducting lines 414 are formed on the first substrate 410, and a counter electrode 422 and metallic conducting lines 424 are formed on the second substrate 420. A dielectric layer 430 is formed on surface of the first substrate 410 so as to cover the surface of the first substrate 410. Therein, the working electrode 412 and the electrode end connector are exposed from the dielectric layer 430 such that the end connector can be electrically connected to the electronic device used for applying electric voltage. An adhesive layer 460 has an empty location formed corresponding to the working electrode 412 and the counter electrode 422. The reactive pad 440 comprising reactive reagent is disposed in the empty location of the adhesive layer 460 and then, the first substrate 410 with the working electrode 412 and the dielectric layer 430 and the second substrate 420 with the counter electrode 422 are attached together face by face, thereby disposing the reactive pad 440 comprising the reactive reagent between the working electrode 412 of the first substrate 410 and the counter electrode 422 of the second substrate 420.

In the present embodiment, the separating pad 450 is disposed between the second substrate 420 and the first substrate 410 at the injection end of blood sample. Preferably, the separating pad 450 is partially exposed so as to facilitate the injection of blood sample. As shown in FIG.4, the blood sample to be detected is injected into the sensing member 400 in direction A, then passes through the separating pad 450 in direction B such that interfering materials of the blood sample can be removed therefrom, and finally the blood sample is laterally diffused to the reactive pad 440 in direction C.

### Embodiment

### Embodiment 1-total cholesterol detection of blood plasma sample

The sensing member of the third embodiment is used to detect total cholesterol of a blood plasma sample. Apply voltage of-330mV on the metallic conducting lines of the working electrode, drip a blood plasma sample of 7µL into the inlet, after about 60 seconds of reaction time, read and record current intensity (µA) and total cholesterol concentration ( mg/dL )in the sample marked by Kodak analyzer. Result is shown in Table 1.

**Table 1**

| | | | |
|---|---|---|---|
| Cholesterol concentration ( mg/dL ) | 121 | 198 | 275 |
| Current intensity (µA) | 1.1 | 2.2 | 4.0 |

### Embodiment 2-total cholesterol detection of whole blood sample

The sensing member of the third embodiment is used to detect total cholesterol of a whole blood sample. Apply voltage of-330mV on the metallic conducting lines of the working electrode, drip a whole blood of 10µL into the inlet, after about 60 seconds of reaction time, read and record current intensity (µA) and total cholesterol concentration ( mg/dL ) in the sample marked by Kodak analyzer. Result is shown in Table 2.

**Table 2**

| | | | | | |
|---|---|---|---|---|---|
| Cholesterol concentration ( mg/dL ) | 116 | 150 | 237 | 262 | 304 |
| Current intensity (µA) | 1.01 | 1.56 | 2.49 | 2.63 | 3.31 |

### Comparative example 1-without using the separating pad

The sensing member of the third embodiment is still used to detect total cholesterol of a whole blood sample. But the separating pad is not disposed in the sensing member according to this example. Apply voltage of-330mV on the metallic conducting lines of the working electrode, drip a whole blood of 10µL into the inlet, after about 60 seconds of reaction time, read and record current intensity (µA) and total cholesterol concentration ( mg/dL ) in the sample marked by Kodak analyzer. The result shows that there is no difference between current signals for whole blood samples respectively containing total cholesterol of 140 mg/dL and 300 mg/dL.

### Comparative example 2-changing position of the separating pad

The sensing member of the third embodiment is still used to detect total cholesterol of a whole blood sample. But the separating pad is disposed between the reactive pad and the second substrate. Apply voltage of -330mV on the metallic conducting lines of the working electrode, drip a whole blood of 10µL into the inlet, after about 60 seconds of reaction time, read and record current intensity (µA) and total cholesterol concentration( mg/dL )in the sample marked by Kodak analyzer. The result shows that there is no difference between current signals for whole blood samples respectively containing total cholesterol of 140 mg/dL and 300 mg/dL.

According to the result in Table 2, current intensity is linearly proportional to total cholesterol of the blood sample. However, in the comparative examples 1 and 2, if the separating pad is not disposed in the sensing member or the position of the separating pad is changed, whole blood samples having total cholesterol of 140 mg/dL to 300 mg/dL cannot be accurately detected. Therefore, the sensing member with the separating pad disposed at specified position according to the present invention can be used to quickly and accurately detect content of total cholesterol of whole blood samples, especially content of total cholesterol of whole blood samples.

The above-described descriptions of the detailed embodiments are only to illustrate the preferred implementation according to the present invention, and it is not to limit the scope of the present invention, Accordingly, all modifications and variations completed by those with ordinary skill in the art should fall within the scope of present invention defined by the appended claims.

## Claims

1. A sensing member for detecting total cholesterol of a whole blood sample, comprising:
a working electrode and a counter electrode respectively formed on a first substrate and a second substrate, wherein an opening is formed in one of the first and second substrates corresponding in position to the electrode for allowing the blood sample to pass through;
a dielectric layer formed on one of the surfaces of the first and second substrates and exposing the electrode and end connector such that the end connector can be electrically connected to an electronic device used for applying electric voltage;
a reactive pad disposed between the working electrode and the counter electrode and comprising an reactive reagent, wherein the reactive reagent comprises an electron transport material, an enzyme capable of reacting with the analyte and generating a current corresponding to the concentration, and a buffer solution;
a separating pad disposed on the opening for removing interfering materials including corpuscles from the blood sample before the blood sample enters into the opening;
a flow conducting sheet disposed between the opening of the electrode and the separating pad, wherein the flow conducting sheet substantially has a same outline as the opening; and
a capillary introducing area disposed above the separating pad.

2. The sensing member of claim 1, wherein the substrates are made of a dielectric polymer.

3. The sensing member of claim 2 wherein the dielectric polymer is at least one of the group consisting of PVC, polystyrene, polyester, polycarbonate, polyether, polyethylene, polypropylene, and PET.

4. The sensing member of claim 1, wherein the substrates are made of one of the group consisting of SiO₂ and AlO₂.

5. The sensing member of claim 1, wherein the electrodes are made of one of the group consisting of carbon electrode, graphite electrode and metal particle.

6. The sensing member of claim 1, wherein the electrodes are formed on surfaces of the substrates through screen print.

7. The sensing member of claim 1, wherein the electron transport material is selected from the group consisting of ferricyanate, ferrocene, para-benzoquinone, phenazine methosulfate, Indophenol, TMB(3,3',5,5'-Tetramethylbenzidine) and β-naphthoquinone-4-potassium sulfonate.

8. The sensing member of claim 7, wherein the ferricyanate is potassium ferricyanide.

9. The sensing member of claim 1, wherein the enzyme capable of reacting with the analyte and generating a current corresponding to the concentration comprises cholesterol esterase, cholesterol oxidase and catalase.

10. The sensing member of claim 1, wherein the buffer solution is one of the group consisting of phosphate buffer solution, TRIS buffer solution and MES buffer solution.

11. The sensing member of claim 1, wherein the reactive reagent further comprises a surface active reagent.

12. The sensing member of claim 1, further comprising an upper covering sheet covering the sensing member.

13. The sensing member of claim 12, wherein the upper covering sheet has an inlet formed corresponding in position to the separating pad for injection of the blood sample.
